(19) Europäisches Patentamt
European Patent Office
Office européen des brevets

(11) Veröffentlichungsnummer: **0 135 142**
**B1**

(12) # EUROPÄISCHE PATENTSCHRIFT

(45) Veröffentlichungstag der Patentschrift: **21.09.88**

(51) Int. Cl.⁴: **C 07 D 501/46,** A 61 K 31/545

(21) Anmeldenummer: **84109661.3**

(22) Anmeldetag: **14.08.84**

(54) **Cephalosporinderivate und Verfahren zu ihrer Herstellung.**

(30) Priorität: **25.08.83 DE 3330605**

(43) Veröffentlichungstag der Anmeldung: **27.03.85 Patentblatt 85/13**

(45) Bekanntmachung des Hinweises auf die Patenterteilung: **21.09.88 Patentblatt 88/38**

(84) Benannte Vertragsstaaten: **AT BE CH DE FR GB IT LI LU NL SE**

(56) Entgegenhaltungen:
**EP-A-0 023 453**
**EP-A-0 045 937**
**FR-A-2 137 899**
**FR-A-2 385 722**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Fleischmann, Klaus, Dr., Winkelgasse 8, D-6500 Mainz 42 (DE)**
Erfinder: **Blumbach, Jürgen, Dr., Manderscheider Strasse 13b, D-6000 Frankfurt am Main 71 (DE)**
Erfinder: **Dürckheimer, Walter, Dr., Im Lerchenfeld 45, D-6234 Hattersheim am Main (DE)**
Erfinder: **Lattrell, Rudolf, Dr., Heuhohlweg 6h, D-6240 Königstein/Taunus (DE)**
Erfinder: **Scheunemann, Karl- Heinz, Dr., Geisenheimer Strasse 88, D-6000 Frankfurt am Main 71 (DE)**
Erfinder: **Klesel, Norbert, Dr., Bornstrasse 50, D-6238 Hofheim am Taunus (DE)**

**EP 0 135 142 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Cephalosporinderivate und Verfahren zu ihrer Herstellung, insbesondere polare Cephemderivate, die in 3-Stellung des Cephemrings durch bestimmte Pyridiniummethyl-Reste substituiert sind, die eine sehr gute antimikrobielle Wirkung gegen gram-positive und gram-negative Bakterien besitzen und deshalb als Arzneimittel zur Behandlung von mikrobiellen Infektionen geeignet sind.

Gegenstand der Erfindung sind daher Cephemderivate der allgemeinen Formel I

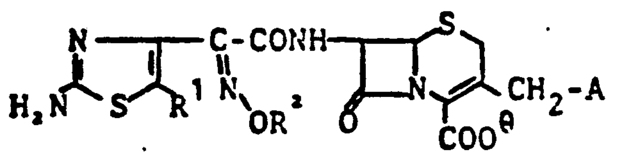

(I)

und ihre physiologisch verträglichen Säureadditionssalze, worin bedeuten

$R^1$ Wasserstoff,

$R^2$ $C_1$-$C_4$-Alkyl, das ein- bis sechsfach substituiert ist durch Fluor,

A einen Pyridiniumrest

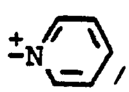

der substituiert sein kann durch $C_3$-$C_6$-Cycloalkyl oder durch 2 orthoständige Alkylgruppen, die zu einem Tri- bis Pentamethylen-Ring geschlossen sind, in dem ein Ring-C-Atom durch ein Sauerstoff- oder Schwefelatom ersetzt sein kann und gleichzeitig mit dem Sauerstoff- oder Schwefelatom auch noch eine Doppelbindung enthalten ist,

und in der die $R^2O$-Gruppe in syn-Position steht.

Der an den Pyridiniumrest ankondensierte Ring kann, wenn man bei der Bezeichnung die beiden benachbarten C-Atome des Pyridiniumrestes mit berücksichtigt, einen Cyclopenteno-, Cyclohexeno- oder Cyclohepteno-Ring darstellen. Als ein Sauerstoffatom enthaltende, ankondensierte Ringe, die eine Doppelbindung enthalten, seien beispielsweise erwähnt Furo und Pyrano, als ankondensierte Ringe mit einem Schwefelatom, die eine Doppelbindung enthalten Thieno und Thiopyrano.

Als besonders bevorzugt kommen beispielsweise die folgenden Substituenten in Betracht:

$R^2$ $C_1$-$C_4$-Alkyl, wie z. B. Methyl, Äthyl, Propyl, Isopropyl, vorzugsweise Methyl und Äthyl, das ein- oder sechsfach, insbesondere 2- bis 4-fach, substituiert ist durch Fluor, wie insbesondere Monofluormethyl, Difluormethyl, Trifluormethyl, 1,1,2,2-Tetrafluoräthyl, 1,1,1-Trifluoräthyl oder 1,1,2,2-Tetrafluorpropyl.

Ist in den vorstehend genannten ankondensierten Ringsystemen ein Ring-C-Atom durch ein Sauerstoff- oder Schwefelatom ersetzt, so kommen insbesondere die folgenden Ringsysteme in Betracht: Furo[2,3-b]pyridin, Furo[3,2-b]pyridin, Furo[2,3-c]pyridin, Furo[3,2-c]pyridin, Thieno[2,3-b]pyridin, Thieno[3,2-b]pyridin, Thieno[2,3-c]pyridin, Thieno[3,2-c]pyridin, Thieno[3,4-b]pyridin, Thieno[3,4-c]pyridin

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Form II

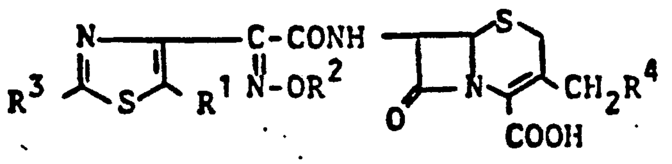

(II)

oder deren Salze oder ein reaktionsfähiges Derivat der Verbindung II, worin $R^1$ und $R^2$ die oben genannte Bedeutung haben und $R^3$ eine Amino- oder eine geschützte Aminogruppe bedeutet und $R^4$ eine durch diejenige Base, die den Resten A der Formel I entsprechen, austauschbare Gruppe bedeutet, mit diesen Basen umsetzt und

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III,

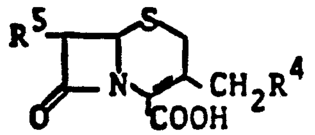

(III)

worin $R^4$ die in Formel II genannte Bedeutung hat und $R^5$ eine Aminogruppe oder eine geschützte Aminogruppe bedeutet, oder deren reaktionsfähige Derivate mit der Base, die dem in Formel I definierten Rest A zugrunde liegt, umsetzt unter Bildung einer Verbindung der allgemeinen Formel IV,

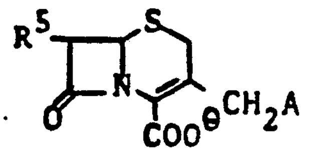

(IV)

in der $R^5$ und A die oben genannte Bedeutung haben,
eine gegebenenfalls vorhandene Aminoschutzgruppe abspaltet und
die Verbindung IV worin $R^5$ eine Aminogruppe bedeutet, entweder als solche oder in Form eines Säureadditionssalzes oder eines reaktionsfähigen Derivates mit einer 2-(2-Aminothiazol-4-yl)-2-syn-oxyiminoessigsäure der allgemeinen Formel V,

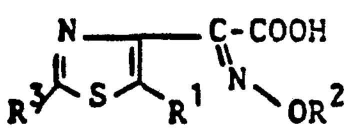

(V)

in der $R^1$, $R^2$ und $R^3$ die oben genannte Bedeutung besitzen, oder mit einem an der Carbonylgruppe aktivierten Derivat dieser Verbindung umsetzt und
α) eine gegebenenfalls vorhandene Schutzgruppe abspaltet und
β) falls erforderlich, das erhaltene Produkt in ein physiologisch verträgliches Säureadditionssalz überführt.

Soll die Herstellung der Verbindungen der allgemeinen Formel I nach dem Verfahren a) durch nucleophilen Austausch von $R^4$ in den Verbindungen der allgemeinen Formel II durch Pyridin oder eines der angegebenen Pyridinderivate erfolgen, so kommen als Reste $R^4$ insbesondere in Betracht Acyloxyrestemit niederen aliphatischen Carbonsäuren, vorzugsweise mit 1 bis 4 C-Atomen, wie z. B.: Acetoxy oder Propionyloxy, insbesondere Acetoxy, die gegebenenfalls substituiert sein können, wie z. B.: Chloracetoxy oder Acetylacetoxy. Für $R^4$ kommen auch andere Gruppen in Betracht, wie beispielsweise Halogen, insbes. Chlor, Brom und Jod, vorzugsweise Jod und Brom, oder Carbamoyloxy.

Erfindungsgemäß werden bei der nucleophilen Austauschreaktion Ausgangsverbindungen der allgemeinen Formel II, in denen $R^4$ für Acetoxy steht, eingesetzt, oder deren Salze, wie z. B. ein Natrium- oder Kaliumsalz. Die Reaktion wird in einem Lösungsmittel, vorzugsweise in Wasser, Formamid oder in einem Gemisch aus Wasser und einem mit Wasser leicht mischbaren organischen Lösungsmittel, wie z. B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Pyridinkomponente wird in Mengen zugegeben, die zwischen etwa äquimolaren Mengen und einem bis zu etwa 5-fachen Überschuß liegt. Der Austausch des Restes $R^4$ wird durch Anwesenheit von Neutralsalzionen, vorzugsweise von Jodid- oder Thiocyanationen, im Reaktionsmedium erleichtert. Insbesondere werden etwa 10 bis etwa 30 Äquivalente Kaliumjodid, Natriumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Liegt die Gruppe $R^3$ als geschützte Aminofunktion vor, so eignen sich als Aminoschutzgruppen z. B. gegebenenfalls substituiertes Alkyl, wie beispielsweise tert.-Butyl, tert.-Amyl; Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z. B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden, z. B. die Tritylgruppe mittels einer Carbonsäure, wie z. B. Essigsäure, Trifluoressigsäure, Ameisensäure, oder die Benzyloxycarbonylgruppe hydrogenolytisch.

Die Reaktionsprodukte der Formel I können aus der Reaktionsmischung in üblicher Weise, z. B. durch Gefriertrocknen der Wasserphase, Chromatographie oder auch durch Ausfällen als schwerlösliches Salz, beispielsweise als Hydrojodid- oder Hydrothiocyanatsalz, isoliert werden.

Die nucleophile Austauschreaktion an Verbindungen der allgemeinen Formel II kann auch so erfolgen, daß

die Reaktion in Gegenwart der den Resten A entsprechenden Basenkomponente und von Tri-$C_1$-$C_4$-alkyljodsilanen, wie z. B. Trimethyl- oder Triäthyljodsilan vorgenommen wird. Diese Variante der Austauschreaktion wird vorteilhafterweise so durchgeführt, daß zu einem Gemisch der Verbindung II und der Base in einem geeigneten Lösungsmittel Trimethyljodsilan zugegeben wird. Es kann aber auch so verfahren werden, daß die Verbindung zuerst mit Trimethyljodsilan nach den unten genannten Reaktionsbedingungen zur Reaktion gebracht wird und sodann die Base zugegeben wird.

Dabei kann beispielsw. so verfahren werden, daß die Verbindung II mit $R^4$ = Acetoxy zuerst mit Trimethyljodsilan nach den im folgenden genannten Reaktionsbedingungen zur Reaktion gebracht wird, die gebildete Verbindung mit $R^4$ = J isoliert und anschließend mit der Base umgesetzt wird, oder die 3-$CH_2$J-Verbindung in situ durch Zugabe der Base zur Reaktion gebracht wird. Eine bevorzugte Ausführungsform ist in den deutschen Patentanmeldungen P 33 16 796, P 33 16 797 und P 33 16 798 beschrieben. Sie besteht darin, daß zu einer Lösung oder Suspension der Verbindung II in einem geeigneten Lösungsmittel die dem Rest A entsprechende Base zugegeben wird, gefolgt von Trimethyljodsilan. Statt Trimethyljodsilan kann beispielsweise auch eine Reaktionsmischung aus Jod und Hexamethyldisilan, die vorher bei Temperaturen zwischen etwa 60 und 120° C in literaturbekannter Weise zur Reaktion gebracht wurde, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.

Geeignete Lösungsmittel sind chlorierte Kohlenwasserstoffe, wie Methylenchlorid, Chlorofom, Dichloräthan, Trichloräthan, Tetrachlorkohlenstoff, oder Niederalkylnitrile, wie Acetonitril oder Propionitril.

Die Base wird in mindestens stöchiometrischer Menge bis zu einem zwanzigfachen Überschuß zugegeben, vorzugsweise wird mit einem fünf- bis fünfzehnfachen Überschuß gearbeitet.

Da neben der auszutauschenden Gruppe $R^4$ in der Ausgangsverbindung II auch andere funktionelle Gruppen, wie z. B. die Carboxylgruppe, mit Trimethyljodsilan reagieren, wird letzteres in mindestens doppeltem bis zu etwa fünfzehn-fachen, vorzugsweise in drei- bis zehnfachem Überschuß zugegeben.

Derartige funktionelle Gruppen können auch durch Zugabe eines Silylierungsmittel wie z. B.: Bistrimethylsilylacetamid, N-Methyl-N-trimethylsilyltrifluoracetamid, Bistrimethylsilyltrifluoracetamid, Trimethylchlorsilan, Hexamethyldisilazan, Bistrimethylsilylharnstoff vorsilyliert werden, entweder ohne oder in Gegenwart einer Base, vorzugsweise der gewünschten der Gruppe A zugrunde liegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Die Reaktion wird bei Temperaturen zwischen -5° C und +100° C, vorzugsweise zwischen 10° C und 80° C ausgeführt.

Die Reaktionsprodukte der Formel I können nach Hydrolyse der Reaktionsmischung durch Zugabe von Wasser oder wässrigen Mineralsäuren, z. B. HCl oder $H_2SO_4$, aus der wässrigen Phase in üblicher Weise, z. B. durch Gefriertrocknen der Wasserphase, Chromatographie und dergleichen isoliert werden. Vorzugsweise werden die polaren Reaktionsprodukte aus der wässrigen Lösung in Form eines schwerlöslichen Salzes, beispielsweise nach Zugabe von KSCN oder KJ, als Hydrothiocyanat- oder Hydrojodidsalz ausgefällt.

Für den Fall, das $R^4$ für eine Carbamoyloxygruppe steht, wird die Austauschreaktion analog durchgeführt.

Steht $R^4$ für Halogen, insbesondere Brom oder Jod, so kann der Austausch in literaturbekannter Weise erfolgen. Liegt die Verbindung II in Form eines reaktionsfähigen Derivates vor, so kommen beispielsweise Silylderivate in Betracht, die bei der Umsetzung von Verbindungen der allgemeinen Formel II mit einer Silylverbindung gebildet werden, wie z. B. Trimethylchlorsilan oder Bis-(trimethylsilyl) acetamid. In diesem Fall wird die Umsetzung zweckmäßig in Gegenwart eines Lösungsmittel, wie Methylenchlorid, Dimethylformamid oder Acetonitril, vorgenommen.

Nach dem Verfahren b) werden die Verbindungen der allgemeinen Formel I durch Acylierung von Verbindungen der allgemeinen Formel IV oder deren Additionssalze, beispielsweise mit Chlorwasserstoffsäure, Bromwasserstoffsäure, Salpetersäure, Schwefelsäure, Phosphorsäure oder einer organischen Säure, wie z. B. Methansulfonsäure oder p-Toluolsulfonsäure mit Carbonsäuren der allgemeinen Formel V oder mit einem reaktionsfähigen Derivat einer solchen Säure durchgeführt.

Es ist dabei nicht erforderlich, die Verbindungen der allgemeinen Formel IV zu isolieren. Die Reaktion kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren reaktionsfähigen Derivate in einem geeigneten Lösungmittel mit der dem Rest A in der allgemeinen Formel IV entsprechenden Base umsetzt und die entstandene Verbindung der allgemeinen Formel IV in situ zu den Verbindungen der allgemeinen Formel I acyliert. Erfindungsgemäß werden bei dieser Reaktion vorzugsweise Ausgangsverbindungen der allgemeinen Formel III, in denen $R^4$ für Jod steht, eingesetzt. Geeignete Lösungsmittel sind chlorierte Kohlenwasserstoffe, wie z. B.: Methylenchlorid und Chloroform, Ether, wie z. B.: Diethylether, Tetrahydrofuran und Dioxan und Amide, wie vorzugsweise Dimethylformamid und Dimethylacetamid. Es kann sich auch als vorteilhaft erweisen, Gemische der genannten Lösungsmittel zu verwenden.

Liegt in den Verbindungen der allgemeinen Formel III ein reaktionsfähiges Derivat vor, so kommen insbesondere Silylderivate in Betracht, die bei der Umsetzung von Verbindungen der allgemeinen Formel III mit Silylverbindungen, wie z. B.: Trimethylchlorsilan oder Bis-(trimethylsilyl)-acetamid gebildet werden. Die dem Rest A entsprechende Base wird in mindestens stöchiometrischer Menge, bis zu einem zehnfachen

Überschuß, vorzugsweise 2 bis 5 Äquivalente verwendet. Die Reaktion wird bei Temperaturen zwischen etwa -5 und +100° C, vorzugsweise zwischen +20 und +50° C ausgeführt.

Die Verbindungen der allgemeinen Formel IV können beispielsweise auch aus Verbindungen der allgemeinen Formel III, wobei $R^4$ für Acetoxy steht, in analoger Weise wie vorstehend für die Verbindungen der allgemeinen Formel II beschrieben, dargestellt werden.

Werden die Carbonsäuren der allgemeinen Formel V sowie ihre an der Aminogruppe geschützten Derivate selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid, gearbeitet.

Die Aktivierung von Carbonsäuren der allgemeinen Formel V kann in besonders günstiger Weise durch Behandlung mit bestimmten Carbonsäureamiden und beispielsweise Phosgen, Phosphorpentachlorid, Tosylchlorid, Thionylchlorid oder Oxalychlorid erfolgen, wie sie z. B. in der deutschen Patentschrift 28 04 040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmittel, wie z. B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel V eignen sich ferner die Anhydride und gemischten Anhydride, Azide, aktivierten Ester und Thioester. Als gemischte Anhydride sind besonders geeignet solche mit niederen Alkansäuren, wie z. B. Essigsäuren und besonders bevorzugt solche mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel V, in denen die Aminogruppe geschützt ist, mit Chlorameisensäurebenzylester, -p-nitrobenzylester, -iso-butylester, -ethylester oder allylester gewinnt.

Als aktivierte Ester eignen sich vorzugsweise diejenigen mit p-Nitrophenol, 2,4-Dinitrophenol, Methylcyanhydrin, N-Hydroxysuccinimid und N-Hydroxyphtalimid, insbesondere diejenigen mit 1-Hydroxybenzotriazol und 6-Chlor-1-hydroxybenzotriazol. Besonders bevorzugte Thioester sind beispielsweise diejenigen mit 2-Mercaptobenzothiazol und 2-Mercaptopyridin. Die aktivierten Derivate können als isolierte Substanzen über auch in situ umgesetzt werden.

Im allgemeinen erfolgt die Umsetzung der Cephemderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z. B. Diäthyläther, vorzugsweise Tetrahydrofuran und Dioxan; Ketone, wie vorzugsweise Aceton und Butanon; Amide, wie vorzugsweise Dimethylformaind und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Die Umsetzung von Cephemverbindungen der Formel IV mit Carbonsäuren der Formel V bzw. deren aktivierten Derivaten kann in einem Temperaturbereich von etwa -80 bis etwa +80° C, vorzugsweise zwischen etwa -20° C und Raumtemperatur erfolgen.

Die Reaktionsdauer hängt von den Reaktanden, der Temperatur und dem Lösungsmittel bzw. dem Lösungsmittelgemisch ab und liegt normalerweise zwischen etwa 1/4 und etwa 72 Std.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z. B. Triäthylamin oder Dimethylanilin, anorganische Basen, wie z. B. Kaliumcarbonat oder Natriumcarbonat, Akylenoxide, wie z. B. Propylenoxid. Auch die Anwesenheit eines Katalysators, wie z. B. von Dimethylaminopyridin, kann gegebenenfalls von Vorteil sein. Liegt in den Verbindungen der allgemeinen Formel IV die Aminogruppe in Form eines reaktionsfähigen Derivats vor, so kann es sich um ein solches handeln, wie aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z. B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z. B. Methylenchlorid, Tetrahydrofuran oder Dimethylformamid durchzuführen.

Als physiologisch verträgliche Säureadditionssalze der Verbindung der allgemeinen Formel I seien beispielsweise erwähnt solche mit Chlorwasserstoffsäure, Bromwasserstoffsäure, Salpetersäure, Phosphorsäure, Schwefelsäure oder organische Säuren, wie z. B. Methansulfonsäure, p-Toloulsulfonsäure oder Maleinsäure.

Die Verbindungen der allgemeinen Formel III und V, sowie die den Pyridiniumresten A entsprechenden Verbindungen sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombinationen mit anderen Wirkstoffen, beispielsweise aus der Reihe der Penicilline, Cephaloporine oder Aminoglykoside zur Anwendung kommen.

Die Verbindungen der allgemeinen Formel I und ihre physiologischen verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit mehreren pharmakologisch verträglichen Trägerstoffen oder Verdünnungsmittel, wie z. B. Füllstoffen, Emulgatoren, Gleitstoffen, Geschmackskorrigentien, Farbstoffen oder Puffersubstanzen vermischt in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seinen beispielsweise erwähnt Traganth, Milchzucker, Talkum, Agar-Agar, Polyglykole, Äthanol und Wasser. Puffersubstanzen sind beispielsweise organische Verbindungen, wie z. B. N,N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen, wie, z. B. Phosphatpuffer, Natriumbicarbonat, Natriumcarbonat. Für die parenterale Applikation kommen vorzugsweise Suspensionen oder Lösungen in Waser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

In der EP-A-0 045 937 werden antimikrobiell wirksame Cephemderivate beschrieben, die eine 2-Aminothiazol-4-yl-Gruppe und einen gegebenenfalls substituierten Pyridiniummethylrest als Substituenten in 3-Stellung des Cephemrings enthalten. Derartige Verbindungen werden auch von den Ansprüchen der EP-A-0 023 453 und FR-A-2 385 722 mit umfaßt.

Die folgenden Ausführungsbeispiele für erfindungsgemäß herstellbare syn-Verbindungen dienen zur weiteren Erläuterung der Erfindung, schränken sie jedoch nicht darauf ein.

**Herstellungsbeispiel 1**

Aktivester der 2-(2-Amino-1,3-thiazol-4-yl)-2-syn-(2,2,2-trifluorethyl)-oxyimino-essigsäure mit 1-Hydroxybenztriazol

3,4 g 2-(2-Amino-1,3-thiazol-4-yl)-2-syn-(2,2,2-trifluorethyl)-oxyimino-essigsäure werden unter Stickstoff in 100 ml trockenem Tetrahydrofuran suspendiert. Nach Zugabe von 3,7 g Dicyclohexylcarbodiimid (DCCI) und 2,5 g 1-hydroxybenztriazol (HOBT) wird 16 Stunden bei Raumtemperatur gerührt. Der ausgefallene Niederschlag wird abgesaugt und das Filtrat i. Vak. eingedampft. Durch Verreiben des öligen Rückstandes mit Diisopropylether erhält man 4,0 g eines farblosen Feststoffes.

**Herstellungsbeispiel 2**

Aktivester der 2-(2-amino-1,3-thiazol-4-yl)-2-syn-difluormethoxyiminoessigsäure mit Hydroxybenztriazol

0,90 g 2-(2-Amino-1,3-thiazol-4-yl)-2-syn-difluormethoxyimino-essigsäure werden unter Stickstoff in 50 ml trockenem Tetrahydrofuran suspendiert. Nach Zugabe von 1,0 g Dicyclohexylcarbodiimid und 0,675 g 1-Hydroxybenztriazol wird 2 Stunden bei Raumtemperatur gerührt. Der ausgefallene Niederschlag wird abfiltriert und das Filtrat i. Vak. eingedampft. Durch Verreiben mit Diisopropylether erhält man 0,90 (67 %) eines farblosen Feststoffes.

**Beispiel 1**

**7-(2-(2-Amino-1,3-thiazol-4-yl)-2-syn-(2,2,2-trifluorethyl)-oxyimino-acetamido)-3-(1-pyridiniomethyl)-ceph-3-em-4-carboxylat**

Verfahren a)

α) 1,50 g 7-(2-(2-Amino-1,3-thiazol-4-yl)-2-syn-(2,2,2-trifluorethyl)-oxyimino-acetamido)-cephalosporansäure werden mit 2,5 ml 1n-NaOH in 20 ml Wasser und 5 ml Aceton gelöst. Man gibt 2,90 g Kaliumrhodanid und 0,50 g

Pyridin zu und rührt unter Stickstoff 3h bei 60° C, wobei der pH-Wert der Lösung durch tropfenweise Zugabe von verdünnter Natriumhydrogencarbonatlösung konstant auf 6,6 gehalten wird. Das Reaktionsgemisch wird anschließend mit Essigester extrahiert, i. Vak. auf ca. 5 ml eingedampft und an Kieselgel mit Aceton/Wasser (3/1) chromatographiert. Die Produktfraktionen werden gefriergetrocknet. Man erhält 0,18 g eines farblosen, amorphen Feststoffes.

IR (KBr): 1770, 1670, 1610 $cm^{-1}$

$^1H$-NMR ($CF_3COOD$): $\delta$ = 3,40 und 3,91 (2H, ABq, J = 18 Hz S-$CH_2$); 4,70 (2H, q, J = 9 Hz ($CH_2$-$CF_3$); 5,45 und 6,23 (2H, ABq, J = 16 Hz, $CH_2$ -N+); 5,39 (1H, d, J = 5 Hz, Lactam-H); 6,05 (1H, d, J = 5 Hz, Lactam-H); 7,39 (1H, s, Thiazol-H); 8,01 - 9,14 (5H, m, Pyridinium-H).

β) Zu einem Gemisch aus 1,50 g 7-(2-(2-Amino-1,3-thiazol-4-yl)2-syn-(2,2,2-trifluorethyl)-oxyimino-acetamido)-cephalosporansäure und 3,6 g Pyridin in 40 ml Methylenchlorid werden unter Kühlung 4,4 g Trimethyljodsilan gegeben und die Mischung 2 Std. unter Rückfluß erhitzt. Nach dem Erkalten wird durch Zugabe von 10 ml 2N HCl hydrolysiert, das Lösungsmittel in Vakuum entfernt und der Rückstand in 20 ml Aceton/Wasser (1 : 1) suspendiert. Von wenig Ungelöstem wird filtriert und das Filtrat über Kieselgel mit Aceton/Wasser (3 : 1) chromatographiert. Die Produktfraktionen werden gefriergetrocknet. Man erhält 0,38 g eines farblosen amorphen Feststoffes. Die Verbindung ist in allen Eigenschaften mit der vorstehend beschriebenen identisch.

Analog Beispiel 1 werden die nachfolgend aufgeführten Verbindungen, die der allgemeinen Formel I mit $R^1$ = Wasserstoff entsprechen und als Reste $R^2$ und A die in Tabelle 1 angegebenen Substituenten tragen als amorphe Feststoffe erhalten.

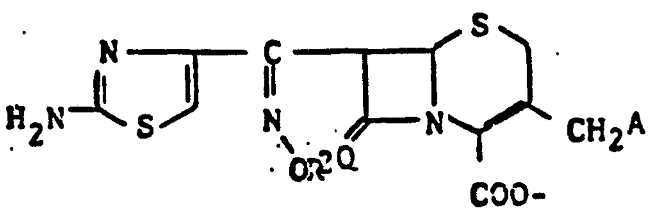

(I')

**Tabelle 1**

| Beispiel | $R^2$ | A | $^1H$-NMR in $CF_3CO_2D$: $\delta$ (ppm) = |
|---|---|---|---|
| 2 | $CH_2CF_3$ | 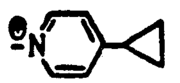 | 1,30 - 1,90 (4H, m, Cyclopropyl-$CH_2$); 2,30 (1H, m, Cyclopropyl CH); 3,37 und 3,87 (2H, ABq, J = 18 Hz, S-$CH_2$); 4,72 (2H, q, J = 9 Hz, $CH_2CF_3$); 5,27 und 6,05 (2H, ABq, J = 16 Hz, $CH_2N$+); 5,41 (1 H, d, J = 5 Hz, Lactam-H); 6,05 (1 H, d, J = 5 Hz, Lactam-H); 7,40 (1 H, s, Thiazol-H); 7,66 (2 H, d, J = 7 Hz, Pyridin-H); 8,70 (2 H, d, J = 7 Hz, Pyridin-H) |
| 3 | $CHF_2$ | 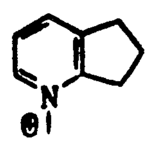 | 2,20 - 2,80 (2 H, m, Cyclopenten-H); 3,10 - 3,80 (6 H, m, 4-Cyclopenten-H und S-$CH_2$); 5,20 - 6,20 (4 H, m, $CH_2$-N+ und 2 Lactam-H); 6,70 (1 H, t, J = 72 Hz, $CHF_2$); 7,45 (1 H, s. Thiazol-H); 7,60 - 8,70 (3 H, m, Pyridin-H); |
| 4 | $CH_2CF_2CF_2H$ | 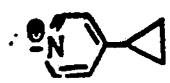 | 1,25 - 1,85 (4 H, m, Cyclopropyl-$CH_2$); 2,30 (1 H, m, Cyclopropyl-CH); 3,35 und 3,85 (2 H, ABq, J = 18 Hz, S-$CH_2$); 4,78 (2 H, t, J = 11 Hz, $CH_2CF_2$); 5,30 und 6,06 (2 H, ABq, J = 16 Hz, $CH_2N$+); |

| | | | |
|---|---|---|---|
| | | | 5,43 (1 H, d, J = 5 Hz, Lactam-H); 6,03 (1 H, d, J = 5 Hz, Lactam-H); 5,88 (1 H, tt, $J_1$ = 50 Hz, $J_2$ = 3 Hz, $CF_2CHF_2$); 7,39 (1 H, s, Thiazol-H); 7,65 (2 H, d, J = 7 Hz, Pyridin-H); 8,68 (2 H, d, J = 7Hz, Pyridin-H); |
| 5 | $CH_2CF_3$ | 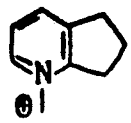 | 2,30 - 3,40 (6 H, m, Cyclopenten-H); 3,37 und 3,83 (2 H, ABq, 18 Hz, $CH_2S$); 4,70 (2 H, q, J = 9 Hz, $CH_2CF_3$); 5,41 und 5,99 (2 H, ABq, J = 16 Hz, $CH_2N+$); 5,45 (1 H, d, J = 5 Hz, Lactam-H); 6,06 (1 H, d, J = 5 Hz, Lactam-H); 7,39 (1 H, s, Thiazol-H); 7,70 - 8,60 (3 H, m, Pyridin-H); |
| 6 | $CH_2CF_2CF_2H$ | 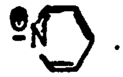 | 3,40 und 3,80 (2 H, ABq, J = 18 Hz, $CH_2S$); 4,78 (2 H, t, J = 11 Hz, $CH_2CF_2$); 5,40 und 6,13 (2 H, ABq, J = 16 Hz, $CH_2N+$); 5,42 (1 H, d, J = 5 Hz, Lactam-H); 6,06 (1 H, d, J = 5 Hz, Lactam-H); 5,85 (1 H, tt, $J_1$ = 50 Hz, $J_2$ = 3 Hz, $CF_2CF_2H$); 7,40 (1 H, s, Thiazol-H); 8,05 - 9,15 (5 H, m, Pyridin-H); |
| 7 | $CH_2CF_2CF_2H$ | 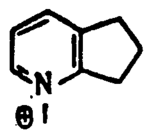 | 2,40 - 3,50 (6 H, m, Cyclopenteno-H); 3,36 und 3,77 (2 H, ABq, J = 18 Hz, S-$CH_2$); 4,81 (2 H, t, J = 11 Hz, $CH_2CH_2$); 5,43 und 6,00 (2 H, ABq, J = 16 Hz, $CH_2N+$); 5,40 (1 H, d, J = 5 Hz, Lactam-H); 5,87 (1 H, tt, $J_1$ = 50 Hz, $J_2$ = 3 Hz, $CF_2CHF_2$); 7,41 (1 H, s, Thiazol-H); 7,70 - 8,65 (3 H, m, Pyridin-H); |
| 8 | $CH_2CF_2CHFCF_3$ | 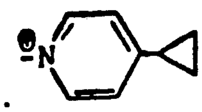 | 1,20 - 1,80 (4 H, m, Cyclopropyl-$CH_2$); 2,20 (1 H, m, Cyclopropyl-CH); 3,33 und 3,83 (2H, ABq, J = 18 Hz, S-$CH_2$); 4,75 (1 H, dt, $J_1$ = 15 Hz, $J_2$ = 2 Hz, $CH_2CF_2$); 5,23 und 6,06 (2 H, ABq, J = 16 Hz, $CH_2N+$); 5,43 (1 H, d, J = 5 Hz, Lactam-H); 6,03 (1 H, d, J = 5 Hz, Lactam-H); 5,85 (1 H, m, $CF_2CHFCF_3$); 7,38 (1 H, s, Thiazol-H); 7,65 (2 H, d, J = 7 Hz, Pyridin-H); 8,69 (2 H, d, J = 7 Hz, Pyridin H); |
| 9 | $CH_2CF_2CHFCF_3$ | 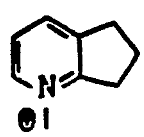 | 2,30 - 3,50 (6 H, m, Cyclopenteno-H); 3,40 und 3,85 (2 H, ABq, J = 18 Hz, S-$CH_2$); 4,79 (2 H, dt, $J_1$ = 15 Hz, $J_2$ = 2 Hz, $CH_2CF_2$); 5,40 und 6,01 (2 H, ABq, J = 15 Hz, $CH_2N+$); 5,45 (1 H, d, J = 5 Hz, Lactam-H); 6,06 (1 H, d, J = 5 Hz, Lactam-H); 5,87 (1 H, m, $CF_2CHF$ $CF_3$); 7,41 (1 H, s, Thiazol-H); 7,60 - 8,60 (3 H, m, Pyridin-H); |

**Beispiel 10**

**7-(2-(2-Amino-1,3-thiazol-4-yl)-2-syn-difluormethoxyamino-acetamido)-3-(1-(2,3-cyclopenteno-pyridinio)-methyl)-ceph-3-em-4-carboxylat**

Verfahren b:

0,85 g 7-Amino-3-(1-(2,3-cyclopenteno-pyridinio)-methyl)-ceph-3-em-4-carboxylat werden unter Stickstoff in 35 ml trockenem Methylenchlorid suspendiert. Nach Zugabe von 1,5 ml N, O- Bis- (trimethylsilyl)-acetamid wird 30 min. bei Raumtemperatur gerührt. Die erhaltene klare Lösung wird im Eisbad gekühlt und 0,78 g des in Herstellungsbeispiel 2 erhaltenen Aktivesters portionsweise zugegeben. Man rührt 12 h bei Raumtemperatur, versetzt mit 100 ml Eiswasser und neutralisiert mit verdünnter Natriumhydrogencarbonatlösung. Die wäßrige Phase wird abgetrennt, zweimal mit Methylenchlorid extrahiert und gefriergetrocknet. Der erhaltene Rückstand wird an Kieselgel mit einem Aceton/Wasser-Gemisch (3/1) chromatographiert. Die Produktfraktionen werden gefriergetrocknet. Man erhält 0,25 g eines farblosen, amorphen Feststoffes, der in allen Eigenschaften mit dem in Beispiel 4 beschriebenen Produkt identisch ist.

Analog Beispiel 10 werden die Verbindungen der Tabelle 2, die der allgemeinen Formel I mit $R^1$ = Wasserstoff entsprechen als amorphe Feststoffe erhalten.

**Tabelle 2**

| Beispiel | $R^2$ | A | $^1$H-NMR in $CF_3CO_2D$: δ (ppm) = |
|---|---|---|---|
| 11 | $CHF_2$ | 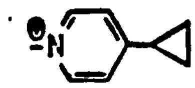 | 1,10 - 1,80 (4 H, m, Cyclopropyl-$CH_2$); 2,30 (1 H, m, Cyclopropyl-CH); 3,35 und 3,90 (2 H, ABq, J = 18 Hz, S-$CH_2$); 5,30 - 6,30 (4 H, m, $CH_2$-N+ und Lactam-H); 6,70 (1 H, t, J = 72 Hz, $CHF_2$); 7,45 (1 H s, Thiazol-H); 7,60 (2 H, d, J = 7 Hz, Pyridin-H); 8,70 (2 H, d, J = 7 Hz, Pyridin-H); |
| 12 | $CHF_2$ | 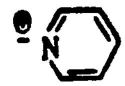 | 3,40 und 3,90 (2 H, ABq, J = 18 Hz, S-$CH_2$); 5,30 - 6,30 (4 H, m, $CH_2$N+ und 2 Lactam-H); 6,70 (1 H, t, J = 72 Hz, $CHF_2$); 7,45 (1 H, s, Thiazol-H); 7,75 - 9,2 (5 H, m, Pyridin-H); |
| 13 | $CHF_2$ | 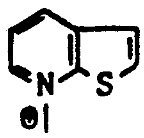 | 3,40 - 3,90 (2 H, ABq, J = 18 Hz, S-$CH_2$); 5,20 - 6,30 (4 H, m, $CH_2$N+ und 2 Lactam-H); 6,70 (1 H, t, J = 72 Hz, $CHF_2$); 7,45 (1 H, s, Thiazol-H); 7,60 - 9,30 (5 H, m, arom. H); |
| 14 | $CH_2CF_3$ | 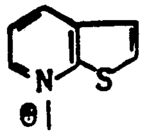 | 3,40 - 3,80 (2 H, ABq, J = 18 Hz, S-$CH_2$); 4,73 (2 H, q, J = 9 Hz, $CH_2CF_3$); 5,20 - 6,50 (4 H, m, $CH_2$N+ und 2 Lactam-H); 7,40 (1 H, s, Thiazol-H); 7,60 - 9,25 (6 H, m, Pyridin-H und Thienyl-H); |

**Beispiel 15**

**7-(2-(2-Amino-1,3-thiazol-4-yl)-2-syn-(2,2,2-trifluor-ethyl)-oxyimino-acetamido)-3-(2,3-cyclohexenopyridinio)-methyl-ceph-3-em-4-carboxylat**

0,68 g 7-Amino-3-jodmethyl-ceph-3-em-4-carbonsäure werden in 40 ml Methylenchlorid suspendiert. Nach Zugabe von 1,5 ml N,O-Bistrimethylsilylacetamid wird 30 min unter Stickstoff, gerührt. Die erhaltene klare Lösung versetzt man mit 0,8 ml 2,3 Cyclohexenopyridin und rührt 3 Stunden. Nach Zugabe von 0,85 g des im Herstellungsbeispiel 1 beschriebenen Aktivesters wird 16 Stunden bei Raumtemperatur gerührt. Man versetzt mit 1 ml Methanol und dampft i. Vak. ein. Der Rückstand wird dreimal mit Diäthyläther verrührt und der Äther abdekantiert. Der erhaltene feste Rückstand wird in 10 ml Aceton/Wasser (3/1) gelöst, mit gesättigter Natriumhydrogencarbonatlösung auf pH = 6 eingestellt und an Kieselgel mit Aceton/Wasser (3/1) chromatografiert. Die Produktfraktionen werden gefriergetrocknet. Man erhält 0,33 g eines farblosen, amorphen Feststoffes.

IR (KBr): 1770, 1670, 1620 $cm^{-1}$
$^1$H-NMR ($CF_3COOD$): δ = 1,8 - 2,3 (4 H, m, Cyclohexeno-H); 2,7 - 3,7 (6 H, m, Cyclohexeno-H und S-$CH_2$);
4,73 (2 H, q, J = 9 Hz, $CH_2CF_3$);
5,65 - 6,2 (4 H, m, 2 Lactam-H und $CH_2$-N+);
7,4 (1 H, s, Thiazol-H); 7,6 - 8,8 g (3 H m Pyridin-H);

Analog Beispiel 15 werden die nachfolgend aufgeführten Verbindungen der Tabelle 3, die der allgemeinen Formel I mit $R^1$ = Wasserstoff entsprechen und als Reste $R^2$ und A die in der Tabelle angegebenen Substituenten tragen als amorphe Feststoffe erhalten.

**Tabelle 3**

| Beispiel | $R^2$ | A | $^1H$-NMR: δ (ppm) in $CF_3CO_2D$ = |
|---|---|---|---|
| 16 | $CH_2CF_3$ | 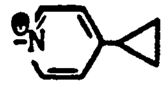 | 1,30 -1,90 (4 H, m, Cyclopropyl-$CH_2$); 2,30 (1 H, m, Cyclopropyl CH); 3,37 und 3,87 (2 H, ABq, J = 18 Hz, S-$CH_2$); 4,72 (2 H, q, J = 9 Hz, $CH_2CF_3$); 5,27 und 6,05 (2 H, ABq, J = 16 Hz, $CH_2N^+$); 5,41 (1 H, d, J = 5 Hz, Lactam-H); 6,05 (1 H, d, J = 5 Hz, Lactam-H); 7,40 (1 H, s, Thiazol-H); 7,66 (2 H, d, J = 7 Hz, Pyridin-H); 8,70 (2 H, d, J = 7 Hz, Pyridin-H) |
| 17 | $CH_2CF_3$ | 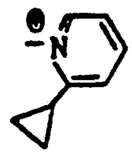 | 1,03 - 1,9 (4 H, m, Cyclopropyl-$CH_2$); 2,1 - 2,65 (1 H, m, Cyclopropyl-CH); 3,2 - 3,9 (2 H, AB, S-$CH_2$, J = 18 Hz); 4,7 (2 H, q, $CH_2$-$CF_3$, J = 8 Hz); 5,33 - 6,4 (4 H, m, β-Lactam und $CH_2$-$N^{\ominus}$); 7,4 (1 H, s, Thiazol-H); 7,63 - 8,95 (4 H, m, Pyridin-H); |

Analog Beispiel 15 werden die nachfolgend aufgeführten Verbindungen der Tabelle 4, die der allgemeinen Formel I mit $R^1$ = Wasserstoff entsprechen und als Reste $R^2$ und A die in der Tabelle angegebenen Substituenten tragen, als amorphe Feststoffe erhalten.

**Tabelle 4**

| Beispiel | $R^2$ | A | $^1H$-NMR: δ (ppm) in $CF_3CO_2D$ = |
|---|---|---|---|
| 18 | $CH_2CH_2F$ | 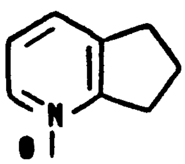 | 2,2-2,7 (2 H, m, Cyclopenten); 3,1 - 4,1 (6 H, m, $SCH_2$ und 4 Cyclopenten-H); 4,3 - 4,5 (2 H, m, $CH_2$); 4,8 - 6,2 (6 H, m, $CH_2F$, $CH_2N^{\oplus}$ und 2 Lactam-H); 7,4 (1 H, s, Thiazol); 7,6 - 8,7 (3 H, m, Pyridin). |

**Patentansprüche** für die Vertragsstaaten: BE, CH, DE, FR, GB, IT, LI, LU, NL, SE

1. Cephemderivate der allgemeinen Formel I

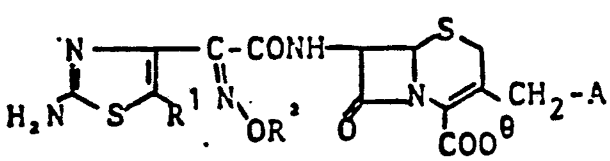 (I).

und ihre physiologisch verträglichen Säureadditionssalze, worin bedeuten

$R^1$ Wasserstoff,

$R^2$ $C_1$-$C_4$-Alkyl, das ein- bis sechsfach substituiert ist durch Fluor,

A einen Pyridiniumrest

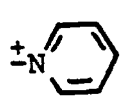,

der substituiert sein kann durch $C_3$-$C_6$-Cycloalkyl oder durch 2 orthoständige Alkylgruppen, die zu einem Tri- bis Pentamethylen-Ring geschlossen sind, in dem ein Ring-C-Atom durch ein Sauerstoff- oder Schwefelatom ersetzt sein kann und gleichzeitig mit dem Sauerstoff- oder Schwefelatom auch noch eine Doppelbindung enthalten ist,

und in der die $R^2O$-Gruppe in syn-Position steht.

2. Verfahren zur Herstellung von Verbindungen der Formel I nach Anspruch 1 und ihrer physiologisch verträglichen Säureadditionssalze, dadurch gekennzeichnet, daß man

a) eine Verbindung der allgemeinen Form II

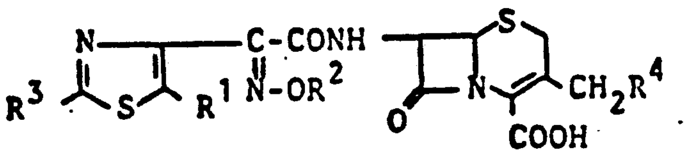

(II)

oder deren Salze oder ein reaktionsfähiges Derivat der Verbindung II worin $R^1$ und $R^2$ die oben genannte Bedeutung haben und $R^3$ eine Amino- oder eine geschützte Aminogruppe bedeutet und $R^4$ eine durch diejenige Base, die den Resten A der Formel I entsprechen, austauschbare Gruppe bedeutet, mit diesen Basen umsetzt und

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III,

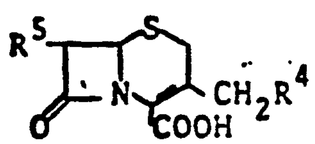

(III)

worin $R^4$ die in Formel II genannte Bedeutung hat und $R^5$ eine Aminogruppe oder eine geschützte Aminogruppe bedeutet, oder deren reaktionsfähige Derivate mit der Base, die dem in Formel I definierten Rest A zugrunde liegt, umsetzt unter Bildung einer Verbindung der allgemeinen Formel IV,

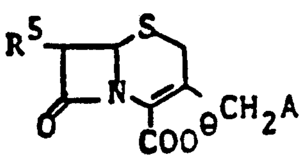

(IV)

in der $R^5$ und A die oben genannte Bedeutung haben,
eine gegebenenfalls vorhandene Aminoschutzgruppe abspaltet und
die Verbindung IV, worin $R^5$ eine Aminogruppe bedeutet, entweder als solche oder in Form eines Säureadditionssalzes oder eines reaktionsfähigen Derivates mit einer 2-(2-Aminothiazol-4-yl)-2-syn-oxyiminoessigsäure der allgemeinen Formel V,

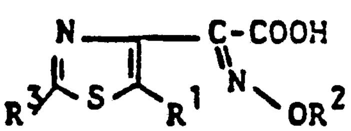

(V)

in der $R^1$, $R^2$ und $R^3$ die oben genannte Bedeutung besitzen, oder mit einem an der Carbonylgruppe aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

3. Verfahren gemäß Anspruch 2, dadurch gekennzeichnet, daß der nucleophile Austausch des Substituenten $R^4$ in Gegenwart von Neutralsalzionen, insbesondere von Jodid oder Thiocyanationen erfolgt.

4. Verfahren gemäß Anspruch 2, dadurch gekennzeichnet, daß der nucleophile Austausch des Substituenten $R^4$ in Gegenwart der dem Rest A zugrundeliegenden Base und von Tri-$C_1$-$C_4$-alkyljodsilan erfolgt.

5. Verfahren nach Anspruch 4, dadurch gekennzeichnet, daß das Tri-$C_1$-$C_4$-alkyljodsilan Trimethyl- oder Triethyljodsilan ist.

6. Gegen bakterielle Infektionen wirksame pharmazeutische Präparate, gekennzeichnet durch einen Gehalt an Cephemderivaten der allgemeinen Formel I.

7. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

8. Cephemderivat der allgemeinen Formel I gemäß Anspruch 1 zur Bekämpfung bakterieller Infektionen.

**Patentansprüche** für den Vertragsstaat: AT

1. Verfahren zur Herstellung von Cephemderivaten der allgemeinen Formel I

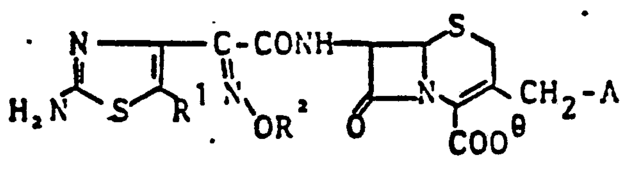

(I)

und ihre physiologisch verträglichen Säureadditionssalzen, worin bedeuten

$R^1$ Wasserstoff,

$R^2$ $C_1$-$C_4$-Alkyl, das ein- bis sechsfach substituiert ist durch Fluor,

A einen Pyridiniumrest

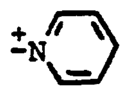 ,

der substituiert sein kann durch $C_3$-$C_6$-Cycloalkyl oder durch 2 orthoständige Alkylgruppen, die zu einem Tri- bis Pentamethylen-Ring geschlossen sind, in dem ein Ring-C-Atom durch ein Sauerstoff- oder Schwefelatom ersetzt sein kann und gleichzeitig mit dem Sauerstoff- oder Schwefelatom auch noch eine Doppelbindung enthalten ist,

und in der die $R^2O$-Gruppe in syn-Position steht,

dadurch gekennzeichnet, daß man

a) eine Verbindung der allgemeinen Form II

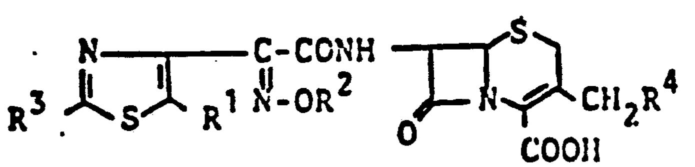

(II)

oder deren Salze oder ein reaktionsfähiges Derivat der Verbindung II, worin $R^1$ und $R^2$ die oben genannte Bedeutung haben und $R^3$ eine Amino- oder eine geschützte Aminogruppe bedeutet und $R^4$ eine durch diejenige Base, die den Resten A der Formel I entsprechen, austauschbare Gruppe bedeutet, mit diesen Basen umsetzt und

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III,

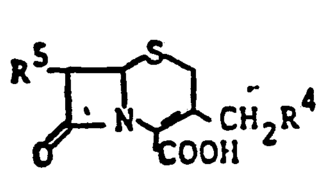

(III)

worin $R^4$ die in Formel II genannte Bedeutung hat und $R^5$ eine Aminogruppe oder eine geschützte Aminogruppe bedeutet, oder deren reaktionsfähige Derivate mit der Base, die dem in Formel I definierten

Rest A zugrunde liegt, umsetzt unter Bildung einer Verbindung der allgemeinen Formel IV,

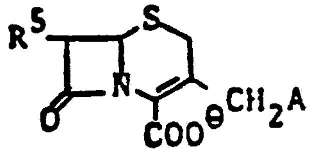 (IV)

in der $R^5$ und A die oben genannte Bedeutung haben,
eine gegebenenfalls vorhandene Aminoschutzgruppe abspaltet und
die Verbindung IV, worin $R^5$ eine Aminogruppe bedeutet, entweder als solche oder in Form eines Säureadditionssalzes oder eines reaktionsfähigen Derivates mit einer 2-(2-Aminothiazol-4-yl)-2-syn-oxyiminoessigsäure der allgemeinen Formel V,

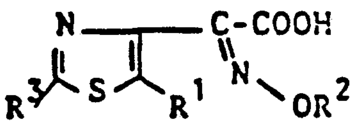 (V)

in der $R^1$, $R^2$ und $R^3$ die oben genannte Bedeutung besitzen, oder mit einem an der Carbonylgruppe aktivierten Derivat dieser Verbindung umsetzt und
α) eine gegebenenfalls vorhandene Schutzgruppe abspaltet und
β) falls erforderlich, das erhaltene Produkt in ein physiologisch verträgliches Säureadditionssalz überführt.

2. Verfahren gemäß Anspruch 1, dadurch gekennzeichnet, daß der nucleophile Austausch des Substituenten $R^4$ in Gegenwart von Neutralsalzionen, insbesondere von Jodid oder Thiocyanationen erfolgt.

3. Verfahren gemäß Anspruch 1, dadurch gekennzeichnet, daß der nucleophile Austausch des Substituenten $R^4$ in Gegenwart der dem Rest A zugrundeliegenden Base und von Tri-$C_1$-$C_4$-alkyljodsilan erfolgt.

4. Verfahren nach Anspruch 3, dadurch gekennzeichnet, daß das Tri-$C_1$-$C_4$-alkyljodsilan Trimethyl- oder Triethyljodsilan ist.

5. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

**Claims** for the Contracting States: BE, CH, DE, FR, GB, IT, LI, LU, NL, SE

1. A cephem derivative of the formula I

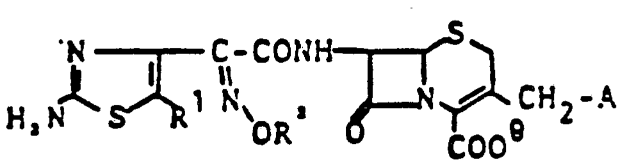 (I)

and its physiologically acceptable acid addition salts, in which

$R^1$denotes hydrogen,

$R^2$denotes $C_1$-$C_4$-alkyl which is monosubstituted to hexasubstituted by fluorine,

A denotes a pyridinium radical,

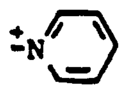

which can be substituted by $C_3$-$C_6$-cycloalkyl or by 2 alkyl groups in ortho positions which are linked to form a trimethylene to pentamethylene ring in which a ring carbon atom can be replaced by an oxygen or sulfur atom and which can, at the same time as the oxygen or sulfur atom, additionally contain a double

bond and in which the $R^2O$ group is in the syn-position.

2. A process for preparing a compound of the formula I as claimed in claim 1 and its physiologically acceptable acid addition salts, which comprises

a) reacting a compound of the formula II

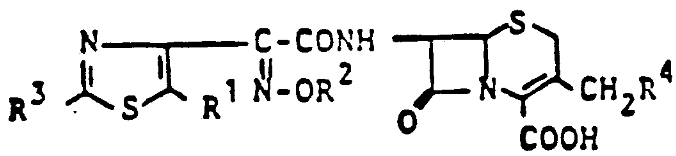

(II)

or its salts or a reactive derivative of the compound II in which $R^1$ and $R^2$ have the abovementioned meanings and $R^3$ denotes an amino or a protected amino group, and $R^4$ denotes a group which can be replaced by those bases which correspond to the radicals A of the formula I, with these bases and

α) splitting off any protective group present and

β) if necessary, converting the product obtained into a physiologically acceptable acid addition salt,

or

b) reacting a compound of the formula III

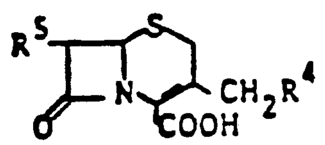

(III)

in which $R^4$ has the meaning mentioned in formula II and $R^5$ denotes an amino group or a protected amino group, or its reactive derivatives, with the base from which the radical A defined in formula I is derived, to form a compound of the formula IV

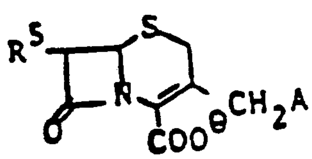

(IV)

in which $R^5$ and A have the abovementioned meanings, splitting off any amino-protecting group present and reacting the compound IV in which $R^5$ denotes an amino group, either as such or in the form of an acid addition salt or of a reactive derivative, with a 2-(2-aminothiazol-4-yl)-2-syn-oxyiminoacetic acid of the formula V

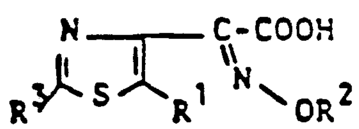

(V)

in which $R^1$, $R^2$ and $R^3$ have the abovementioned meanings, or with a derivative of this compound which is activated at the carbonyl group, and

α) splitting off any protective group present and

β) if necessary, converting the product obtained into a physiologically acceptable acid addition salt.

3. The process as claimed in claim 2, wherein the nucleophilic replacement of substituent $R^4$ takes place in the presence of neutral salt ions, in particular iodide or thiocyanate ions.

4. The process as claimed in claim 2, wherein the nucleophilic replacement of substituent $R^4$ takes place in the presence of the parent base of radical A and in the presence of tri-$C_1$-$C_4$-alkyliodosilane.

5. The process as claimed in claim 4, wherein the tri-$C_1$-$C_4$-alkyliodosilane is trimethyliodosilane or triethyliodosilane.

6. Pharmaceutical preparations active against bacterial infections, which contain a cephem derivative of the formula I.

7. A process for preparing pharmaceutical preparations active against bacterial infections, which comprises bringing a cephem derivative of the formula I, if appropriate together with pharmaceutically customary excipients or diluents, into a pharmaceutically suitable administration form.

8. Use of a cephem derivative of the formula I, for fighting bacterial infection.

**Claims** for the Contracting State: AT

1. A process for preparing a cephem derivative of the formula I

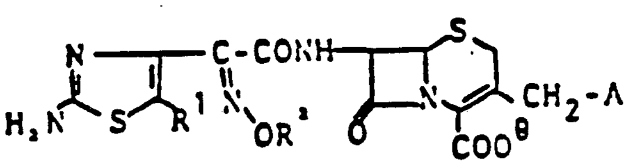

(I)

and its physiologically acceptable acid addition salts, in which

$R^1$ denotes hydrogen,

$R^2$ denotes $C_1$-$C_6$-alkyl which is monosubstituted to hexa-substituted by fluorine,

A denotes a pyridinium radical,

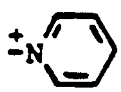

which can be substituted by $C_3$-$C_6$-cycloalkyl, or by 2 alkyl groups in ortho positions which are linked to form a trimethylene to pentamethylene ring in which a ring carbon atom can be replaced by an oxygen or sulfur atom and which can, at the same time as the oxygen or sulfur atom, additionally contain a double bond and in which the $R^2O$ group is in the syn-position,

which comprises

a) reacting a compound of the formula II

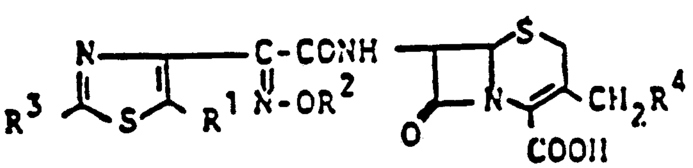

(II)

or its salts or a reactive derivative of the compound II in which $R^1$ and $R^2$ have the abovementioned meanings and $R^3$ denotes an amino or a protected amino group, and $R^4$ denotes a group which can be replaced by those bases which correspond to the radicals A of the formula I, with these bases and

α) splitting off any protective group present and

β) if necessary, converting the product obtained into a physiologically acceptable acid addition salt,

or

b) reacting a compound of the formula III

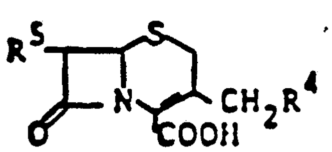

(III)

in which $R^4$ has the meaning mentioned in formula II and $R^5$ denotes an amino group or a protected amino group, or its reactive derivatives, with the base from which the radical A defined in formula I is derived, to form a compound of the formula IV

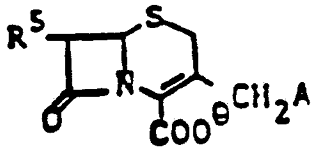

(IV)

in which $R^5$ and A have the abovementioned meanings, splitting off any amino-protecting group present and reacting the compound IV in which $R^5$ denotes an amino group, either as such or in the form of an acid addition salt or of a reactive derivative, with a 2-(2-aminothiazol-4-yl)-2-syn-oxyiminoacetic acid of the formula V

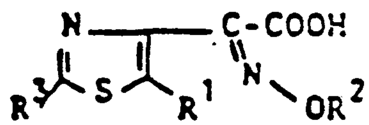

V

in which $R^1$, $R^2$ and $R^3$ have the abovementioned meanings, or with a derivative of this compound which is activated at the carbonyl group, and

α) splitting off any protective group present and

β) if necessary, converting the product obtained into a physiologically acceptable acid addition salt.

2. The process as claimed in claim 1, wherein the nucleophilic replacement of substituent $R^4$ takes place in the presence of neutral salt ions, in particular iodide or thiocyanate ions.

3. The process as claimed in claim 1, wherein the nucleophilic replacement of substituent $R^4$ takes place in the presence of the parent base of radical A and in the presence of tri-$C_1$-$C_4$-alkyliodosilane.

4. The process as claimed in claim 3, wherein the tri-$C_1$-$C_4$-alkyliodosilane is trimethyliodosilane or triethyliodosilane.

5. A process for preparing pharmaceutical preparations active against bacterial infections, which comprises bringing a cephem derivative of the formula I, if appropriate together with pharmaceutically customary excipients or diluents, into a pharmaceutically suitable administration form.

**Revendications** pour les Etats Contractants: BE, CH, DE, FR, GB, IT, LI, LU, NL, SE

1. Dérivés céphème de formule générale I

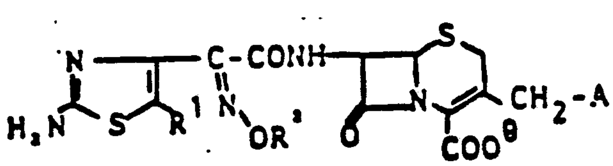

(I)

et sels d'addition de ceux-ci avec des acides physiologiquement acceptables, dans laquelle formule

$R^1$représente un atome d'hydrogène,

$R^2$représente un groupe alkyle en $C_1$-$C_4$ qui est substitué de 1 à 6 fois par des atomes de fluor,

A représente un reste pyridinium

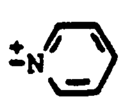

peut être substitué par un groupe cycloalkyle en $C_3$-$C_6$ ou par deux groupes alkyle en position ortho qui sont cyclisés en un cycle tri- à pentaméthylène, dans lequel un atome de carbone nucléaire peut être remplacé par un atome d'oxygène ou de soufre, et en outre une double liaison est contenue en même temps que l'atome d'oxygène ou de soufre,

et dans laquelle le groupe O-$R^2$ est en position syn.

2. Procédé pour la préparation de composés de formule I selon la revendication 1, et de leurs sels d'addition avec des acides physiologiquement acceptables, caractérisé en ce que

a) on fait réagir un composé de formule II

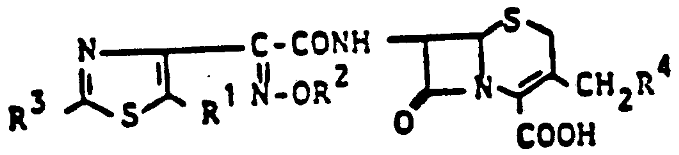

(II)

ou un de ses sels, ou un dérivé réactif du composé II, dans lequel $R^1$ et $R^2$ ont les significations données plus haut, et $R^3$ représente un groupe amino ou un groupe amino protégé, et $R^4$ représente un groupe remplaçable par la base à laquelle correspondent les restes A de la formule I, avec cette base, et

α) on élimine un groupement protecteur éventuellement présent, et

β) si nécessaire, on convertit le produit obtenu en un sel d'addition avec un acide physiologiquement acceptable,

ou

b) on fait réagir un composé de formule générale III

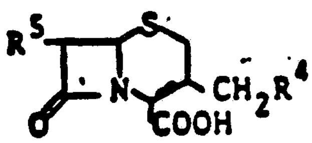

(III)

dans laquelle $R^4$ a la signification donnée dans la formule II et $R^5$ représente un groupe amino ou un groupe amino protégé, ou des dérivés réactifs de celui-ci, avec la base à laquelle correspond le reste A défini dans la formule I, avec formation d'un composé de formule générale IV

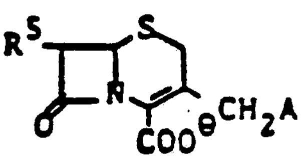

(IV)

dans laquelle $R^5$ et A ont les significations données plus haut, on élimine un groupement protecteur de groupe amino éventuellement présent, et

on fait réagir le composé IV, dans lequel $R^5$ représente un groupe amino, soit tel quel soit sous forme d'un sel d'addition avec un acide, soit d'un dérivé réactif, avec un acide 2-(2-aminothiazole-4-yl)-2-syn-oxyiminoacétique de formule générale V

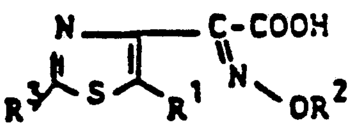

(V)

dans laquelle $R^1$, $R^2$ et $R^3$ ont les significations données plus haut, ou avec un dérivé de ce composé activé au niveau du groupe carbonyle, et

α) on élimine un groupement protecteur éventuellement présent, et

β) si nécessaire, on convertit le produit obtenu en un sel d'addition avec un acide physiologiquement acceptable.

3. Procédé selon la revendication 2, caractérisé en ce que l'on effectue l'échange nucléophile du substituant $R^4$ en présence d'ions de sels neutres, en particulier d'ions iodure ou thiocyanate.

4. Procédé selon la revendication 2, caractérisé en ce que l'on effectue l'échange nucléophile du substituant $R^4$ en présence de la base de laquelle dérive le reste A, et d'un trialkyl($C_1$-$C_4$)-iodosilane.

5. Procédé selon la revendication 4, caractérisé en ce que le trialkyl($C_1$-$C_4$)-iodosilane est le triméthyl- ou triéthyl-iodosilane.

6. Composition pharmaceutique active contre des infections bactériennes, caractérisée par une teneur en dérivés céphème de formule générale I.

7. Procédé de préparation de compositions pharmaceutiques actives contre des infections bactériennes, caractérisé en ce que l'on met sous une forme pharmaceutiquement appropriée un dérivé céphème de formule générale I, éventuellement avec des véhicules ou diluants pharmaceutiques usuels.

8. Dérivé céphème de formule générale I selon la revendication 1, pour la lutte contre des infections bactériennes.

**Revendications** pour l'Etat Contractant: AT

1. Procédé pour la préparation de dérivés céphème de formule générale I

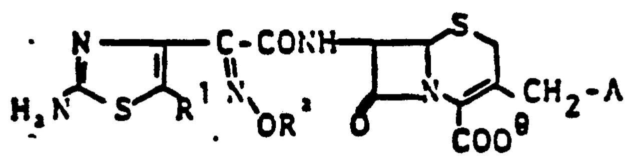

(I)

et de leurs sels d'addition avec des acides physiologiquement acceptables, dans laquelle formule

$R^1$représente un atome d'hydrogène,

$R^2$représente un groupe alkyle en $C_1$-$C_4$ qui est substitué de 1 à 6 fois par des atomes de fluor,

A représente un reste pyridinium

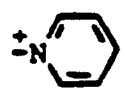

qui peut être substitué par un groupe cycloalkyle en $C_3$-$C_6$ ou par deux groupes alkyle en position ortho qui sont cyclisés en un cycle tri- à pentaméthylène, dans lequel un atome de carbone nucléaire peut être remplacé par un atome d'oxygène ou de soufre, et en outre une double liaison est contenue en même temps que l'atome d'oxygène ou de soufre,

et dans laquelle le groupe O-$R^2$ est en position syn,

caractérisé en ce que

a) on fait réagir un composé de formule II

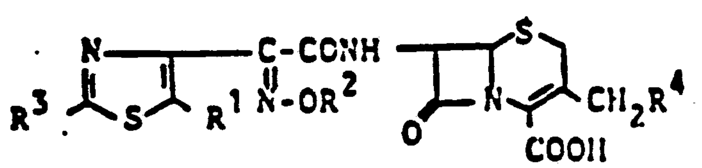

(II)

ou un de ses sels, ou un dérivé réactif du composé II, dans lequel $R^1$ et $R^2$ ont les significations données plus haut, et $R^3$ représente un groupe amino ou un groupe amino protégé, et $R^4$ représente un groupe remplaçable par la base à laquelle correspondent les restes A de la formule I, avec cette base, et

α) on élimine un groupement protecteur éventuellement présent, et

β) si nécessaire, on convertit le produit obtenu en un sel d'addition avec un acide physiologiquement acceptable,

ou

b) on fait réagir un composé de formule générale III

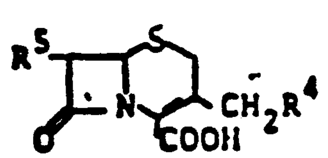

(III)

dans laquelle $R^4$ a la signification donnée dans la formule II et $R^5$ représente un groupe amino ou un groupe amino protégé, ou des dérivés réactifs de celui-ci, avec la base à laquelle correspond le reste A défini dans la formule I, avec formation d'un composé de formule générale IV

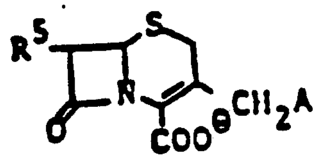

(IV)

dans laquelle $R^5$ et A ont les significations données plus haut, on élimine un groupement protecteur de groupe amino éventuellement présent, et
on fait réagir le composé IV, dans lequel $R^5$ représente un groupe amino, soit tel quel soit sous forme d'un sel d'addition avec un acide, soit d'un dérivé réactif, avec un acide 2-(2-aminothiazole-4-yl)-2-syn-oxyiminoacétique de formule générale V

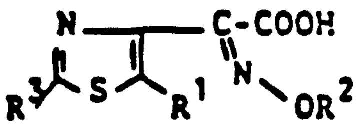

(V)

dans laquelle $R^1$, $R^2$ et $R^3$ ont les significations données plus haut, ou avec un dérivé de ce composé activé au niveau du groupe carbonyle, et

α) on élimine un groupement protecteur éventuellement présent, et

β) si nécessaire, on convertit le produit obtenu en un sel d'addition avec un acide physiologiquement acceptable.

2. Procédé selon la revendication 1, caractérisé en ce que l'on effectue l'échange nucléophile du substituant $R^4$ en présence d'ions de sels neutres, en particulier d'ions iodure ou thiocyanate.

3. Procédé selon la revendication 1, caractérisé en ce que l'on effectue l'échange nucléophile du substituant $R^4$ en présence de la base de laquelle dérive le reste A, et d'un trialkyl($C_1$-$C_4$)-iodosilane.

4. Procédé selon la revendication 3, caractérisé en ce que le trialkyl($C_1$-$C_4$)-iodosilane est le triméthyl- ou triéthyl-iodosilane.

5. Procédé de préparation de compositions pharmaceutiques actives contre des infections bactériennes, caractérisé en ce que l'on met sous une forme pharmaceutiquement appropriée un dérivé céphème de formule générale I, éventuellement avec des véhicules ou diluants pharmaceutiques usuels.